**Europäisches Patentamt**

(19) **European Patent Office**

**Office européen des brevets**

(11) Publication number : **0 350 098 B1**

(12) **EUROPEAN PATENT SPECIFICATION**

(45) Date of publication of patent specification :
24.03.93 Bulletin 93/12

(51) Int. Cl.⁵ : **C12Q 1/34**

(21) Application number : **89201699.9**

(22) Date of filing : **28.06.89**

(54) **Method for evaluating detergent cellulases.**

(30) Priority : **05.07.88 GB 8815975**

(43) Date of publication of application :
**10.01.90 Bulletin 90/02**

(45) Publication of the grant of the patent :
**24.03.93 Bulletin 93/12**

(84) Designated Contracting States :
**AT BE CH DE ES FR GB GR IT LI LU NL SE**

(56) References cited :
**EP-A- 0 244 932**
**GB-A- 2 075 028**
**TEXTILE RESEARCH JOURNAL, June 1959,**
**Lancaster, PA (US); J.W.HENSLEY et al., pp.**
**505-513**

(56) References cited :
**THE JOURNAL OF THE AMERICAN OIL**
**CHEMISTS' SOCIETY, vol. 43, 1966, Chicago,**
**IL (US); B.E.GORDON et al., pp. 525-528**
**PURE AND APPLIED CHEMISTRY, vol. 59, no.**
**2, 1987, Oxford (GB); T.K.GHOSE, pp. 257-268**

(73) Proprietor : **THE PROCTER & GAMBLE**
**COMPANY**
**One Procter & Gamble Plaza**
**Cincinnati Ohio 45202 (US)**

(72) Inventor : **Barrat, Christian Roland**
**Avenue Wannecouter 38**
**B-1020 Brussels (BE)**

(74) Representative : **Canonici, Jean-Jacques et al**
**Procter & Gamble European Technical Center**
**N.V. Temselaan 100**
**B-1853 Strombeek-Bever (BE)**

EP 0 350 098 B1

## Description

TECHNICAL FIELD

The invention relates to an efficient, performance-predictive, analytical method for evaluating the performance of cellulase preparations for use in detergent compositions.

The method utilizes the cellulase action on a radiolabelled substituted cellulosic material deposited onto a fabric substrate.

BACKGROUND OF THE INVENTION

Since their contribution to detergency was discovered, enzymes have become well-known components of detergent compositions, and attention of detergent as well as enzyme manufacturers is constantly focused on the development of enzymes which exhibit an improved cleaning performance and/or provide additional benefits to the treated fabrics.

Cellulase enzymes have been found to be particularly effective species for use in fabric-treatment compositions, since they exhibit both cleaning and fabric-softening properties; particularly remarkable is their softening through-the-wash effect on cotton fabrics upon cumulative washes.

Enzyme manufacturers generally use analytical methods, for the determination of cellulase activities, while detergent manufacturers have applied standard non-analytical methods to quantify the performance of cellulases in detergent compositions.

Said analytical methods have been described in various publications, such as : "Measurement of cellulase activities" - T.K. Ghose - Pure & Appl. chem. vol. 59 n° 2 p. 257-268 (1987).

Such known analytical methods typically used by enzyme manufacturers generally measure the cellulase action on a soluble substituted cellulose substrate, which is generally carboxy methyl cellulose. Cellulase activity can be assayed by measuring the increase in reducing sugar formation, after hydrolysis of solubilized carboxy methyl cellulose; In US-B-4,435,307 is described such a method.

Another known method consists in measuring the viscosity change of the solution, after hydrolysis of solubilized carboxy methyl cellulose. In GB-B-1 368 594 is described such a method.

However, when applied to a laundry detergent context, the above analytical methods to determine cellulase activity have not been found to correlate with the actual performance of the enzyme, particularly in terms of fabric softening upon cumulative washes; this can be due to the interference of detergent ingredients such as surface-active agents; another reason is that most commercial cellulase preparations have multiple cellulolytic activities, which are not well discriminated by the above analytical methods, while, in fact, it is far from certain that all activities are performing in a laundry context. For instance, cellobiohydrolase which is a known component of cellulase preparations, does not exhibit fabric softening performance, but does react in analytical methods, based on sugar reducing groups formation with carboxy methyl cellulose.

On the other hand, the standard methods typically used by detergent manufacturers to evaluate e.g. softening performance, frequently using panel evaluations, are subjective, and less accurate than any analytical method; more importantly they require relatively high quantities of material, and a substantial amount of time, especially when a multi-wash procedure needs to be followed, which is the case in evaluating cotton softness.

There is a need, therefore, for an efficient, performance-predictive, analytical method to evaluate the performance of cellulase preparations for use in fabric-treatment compositions.

The method of the invention answers the above need; it provides an analytical method, perfectly adapted to the evaluation of detergent cellulases; the present method uses a substituted cellulosic material, which unlike in known analytical methods, is rendered insoluble by deposition onto a fabric substrate.

Said substituted cellulosic material is labelled with a radioactive isotope. Carboxy-methyl-cellulose labelling by carbon 14 is known; it has been used to evaluate the soil antiredeposition properties of carboxy-methyl-cellulose, in : "Absorption of Labelled Sodium Carboxy Methyl Cellulose by Textile Fibers" J.W. Hensley and C.G. Inks, Textile Research Journal, June 1959, p 505-513.

It is the object of the present invention to provide an efficient, performance-predictive, analytical method specifically adapted to the evaluation of cellulase preparations for use in detergent compositions.

SUMMARY OF THE INVENTION

The present invention relates to a method for evaluating the performance of cellulase preparations for use in detergent compositions, comprising the steps of :
- depositing a radiolabelled substituted cellulosic material onto a fabric substrate.

- washing said fabric substrate with a composition containing the cellulase preparation to be evaluated.
- determining, by radioactivity measurement, the amount of substituted cellulosic material removed from said fabric substrate, by the cellulase.

Preferably, the composition containing the cellulase preparation to be evaluated, is a detergent composition, and the radiolabelled substituted cellulosic material is carboxy methyl cellulose radiolabelled with carbon 14.

## DETAILED DESCRIPTION OF THE INVENTION

The first step of the present method consists in the deposition of a radiolabelled substituted cellulosic material onto a fabric substrate.

The fabric substrate is preferably represented by one or more swatches of fabric of uniform predetermined size, the fabric being most preferably a cellulosic fabric, in particular cotton.

The substituted cellulosic material can be carboxy methyl cellulose, or C1-C4 alkyl cellulose ether or any functionalized cellulosic polymer; carboxy methyl cellulose is the preferred material.

Carboxy methyl cellulose (CMC) is a well-known ingredient in the detergent art. It is preferably used as a salt, with conventional cations such as sodium, potassium, amines or substituted amines.

The degree of substitution of CMC (DS) ranges from 0.3 to 1,5, preferably from 0.6 to 0.9, and the degree of polymerisation (DP) in the ranges from 50 to 1000, preferably from 200 to 400.

A typical example of a suitable CMC for use herein is the sodium salt having a D.S. of about 0.7 and a D.P. of about 250.

Radiolabelling of the substituted cellulosic material is effected by state-of-the art methods, using preferably carbon 14, although other radioactive isotopes can be used such as tritium.

A preferred material is a sodium salt of carboxy methyl cellulose with a carbon 14 labelling on the carboxy group as prepared according to the Hensley & Inks publication referred to herein above.

A precisely known amount of an aqueous solution of the radiolabelled material is volumetrically applied onto the fabric substrate, in an amount not exceeding what can be naturally absorbed by the fabric. The fabric substrate is allowed to dry.

Preferably, the dry fabric-substrate containing the radiolabelled material is then incubated in hard water in order to consolidate the fixation of the radiolabelled material onto the substrate.

Then, the fabric substrate containing the radiolabelled substituted cellulosic material is washed with a composition containing the cellulase preparation to be evaluated. Such a composition may simply be an aqueous solution of the cellulase preparation, buffered at the pH which will be applicable in a detergent context. Preferably, however, the composition is a detergent composition, in granular or liquid form, and the washing is conducting according to standard detergent-testing method, with optimized variables as wash water hardness, temperature, cycle duration and composition concentration.

The radioactivity of the fabric substrate containing the radiolabelled substituted cellulosic material is measured before and after the washing step; the comparison between the results permits to calculate the amount of substituted radiolabelled cellulosic material removed from the fabric substrate.

When the preferred incubation step in hard water has occured, radioactivity of the unwashed substrate is measured after incubation. To calculate the percentage of removal of the radiolabelled substituted cellulosic material, by the cellulase only, the composition "matrix" effect is taken into account; this is most conveniently done by measuring the radioactivity of the fabric substrate washed with the same composition as above, but not containing any cellulase.

Radioactivity measurements most conveniently use scintillation counting techniques selected in accordance to the type of labelling; the beta energy counting procedure, useful with carbon 14 labelling, is described, for instance, in "Liquid Scintillation Determination of Surfactants on Cotton Fabrics" B.E. Gordon JAOCS 1966 (43) p.525.

## Applications of the method of the invention

The method of the invention will advantageously be used for the following applications :
- screening of cellulase preparations with a view to use them in laundry detergent compositions; the present method will especially be useful to measure the efficiency of new, improved cellulases in terms of fabric softening performance, in comparison to known and commercially available ones.
- study of cellulase stability upon storage, in a detergent context; calibration with a known level of fresh cellulase is used, to evaluate the residual level of cellulase in the aged composition
- quality control of detergent compositions containing cellulases.

EP 0 350 098 B1

EXPERIMENTAL PART

NB : $C_{14}$ CMC is used to designate Carboxy Methyl Cellulose radiolabelled on the carboxy group with Carbon 14.

EXAMPLE 1

The method of the invention is used to comparatively evaluate the fabric softening performance of two commercially available cellulase preparations : celluzyme [R] (supplier activity : 1380 CMCU/g); and Celluclast [R] (supplier activity : 1680 NCU/g) both supplied by NOVO INDUSTRI A/S (Denmark).

Three types of detergent compositions are prepared, all based on heavy duty liquid detergent composition commercially available under the trade name VIZIR.

Such a liquid detergent composition typically contains the following ingredients :

| ingredient | % by weight |
|---|---|
| Linear $C_{12}$ alkyl benzene sulphonic acid | 11.0 |
| Coconut alkyl sulphate (TEA salt) | 4.0 |
| $C_{12-15}$ alcohol ethoxylate (E07) | 10.0 |
| Coconut fatty acid | 10.0 |
| Oleic acid | 5.0 |
| Citric acid | 1.0 |
| Triethanolamine | 4.0 |
| Ethanol | 6.0 |
| Propanediol | 1.5 |
| Sodium hydroxide | 3.0 |
| Sodium formate | 1.0 |
| Protease | 0.6 |
| minors, water | balance to 100 |

One type of detergent composition does not contain any cellulase (reference composition), the second type contains Celluzyme [R] at a level of 3.3 % by weight of the total composition, the third type contains Celluclast [R] at a level of 3.0 % by weight of the total composition. The difference in levels for the two different cellulases compensates for the difference in their activities, so that the total cellulolytic activity (based on reducing sugar formation) in the detergent compositions containing Celluzyme [R] is equal to the total cellulolytic activity in the detergent compositions containing Celluclast [R].

Separately, a stock solution of $C_{14}$ CMC is prepared; $C_{14}$ CMC can be obtained by methods described in the literature, as referred to hereinabove.

The characteristics of the $C_{14}$ CMC are as follows :
    radioactivity : 0.7 millicurie/g
    DS : about 0.7; DP : about 250.
The stock solution is prepared by dissolving 0,026 g of $C_{14}$ CMC and 0,1726 g of detergent-grade CMC in 170 g of deionized water and 30 g of ethanol.

The procedure is as follows :

A series of 13 muslin cotton swatches, all measuring 5 cm x 5 cm, is prepared; at the center of each swatch are deposited exactly 350 microliters of the stock solution of $C_{14}$ CMC. After air drying, the swatches are all put in a launderometer jar filled with 400 ml of hard water (city water) and incubated in a heat up cycle, from 20° C to 60° C in 40 minutes.

4

The swatches are then rinsed in a beaker under running tap water for one minute; they are then squeezed and allowed to air-dry for at least 30 minutes.

At this stage, four of the swatches are kept as reference i.e. are not taken through the washing step which follows.

The remaining nine swatches are put in individual 20 cc glass vials. The detergent compositions prepared as seen hereinabove are used to form aqueous solutions at 1% detergent composition concentration (4 mmole/l hardness) in order to create realistic washing machine conditions.

Three treatments are then conducted, each treatment consisting of washing a swatch with one of the three types of detergent compositions prepared hereinabove. For each treatment, three replicates are used, for better accuracy. For this purpose, three of the vials are filled with 10 ml of a solution of detergent composition containing no cellulase, three other vials are filled with the same amount of a solution of detergent composition containing Celluzyme [(R)], while the three remaining vials are filled with the same amount of a solution of detergent composition containing Celluclast [(R)].

The filled vials are put in launderometer jars filled with soft water (maximum 5 vials per jar); the launderometer jars are put in the launderometer type equipment (Linitest Original Hanau) for washing in a heat-up cycle, from 20° C to 60° C in 40 minutes.

After cooling of the vials, the swatches are taken out of their respective vials, rinsed in a beaker under running soft water, and allowed to dry.

The unwashed (reference) swatches, as well as the swatches which have just undergone the washing step, are put each in plastic vials filled with 12 ml of scintillator liquid (scintillator 299 from Packard), which are allowed to stabilize for at least 30 minutes.

All the vials are put in a LKB 1210 Ultrobeta Scintillation Counter at optimum operating conditions; the radioactivity counts for each swatch is thus obtained.

The percentage of $C_{14}$ CMC removal (% $C_{14}$ CMC removal) can be calculated as follows : % $C_{14}$ CMC removal $= \dfrac{Y - X}{Y} \times 100$

wherein

Y is the radioactivity count on unwashed swatch

X is the radioactivity count on washed swatch.

The percentage of $C_{14}$ CMC removal by the cellulase action only (i.e. taking into account the detergent matrix effect) is calculated as follows % $C_{14}$ CMC removal by cellulase $= \dfrac{T - Z}{100 - Z} \times 100$

wherein

T is % $C_{14}$ CMC removal by the detergent composition containing cellulase

Z is % $C_{14}$ CMC removal by the detergent composition not containing cellulase.

The results are as follows : (1)

% $C_{14}$ CMC removal by cellulase in :

| detergent composition without cellulase (reference) | detergent composition with Celluzyme [(R)] | detergent composition with Celluclast [(R)] |
|---|---|---|
| O | 30** | 19* |

* significant difference versus reference

** significant difference versus both reference and the other cellulase treatment

Separately, through-the-wash fabric softness is evaluated by a standard full-fledged washing machine, for the same detergent compositions containing Celluzyme [(R)] and Celluclast [(R)] enzymes.

The design of the test is such as to compare softeness of textile pieces (housewife harsh terry towels) laundered cumulatively 4 times and then 8 times, between the compositions to be tested and a reference composition, which is the same detergent composition, not containing any cellulase. Each towel is cut in 3 identical parts, one for each of the three treatments conducted (reference, Celluzyme [(R)], Celluclast [(R)]); 8 towels are used (8 replicates).

The testing conditions are as follows :
- automatic drum washing machine MIELE® 423
- washing cycle up to 60°C
- normally soiled ballast loads (2.5 kg) comparable in the 3 treatments
- 1% product concentration in wash liquor
- water hardness : 2.5 mmole/l.

The washed and line dried test terry towels are compared by a panel of two expert judges, working independently, by a paired comparison grading technique using a 9-point Schaeffe scale. Differences are recorded in panel score units (psu), positive being performancewise better than the reference treatment.

The testing results are as follows : (2)

## Softness performance of cellulase in :

|  | detergent composition without cellulase (reference) | detergent composition with Celluzyme [(R)] | detergent composition with Celluclast [(R)] |
|---|---|---|---|
| after 4 washes | 0 | + 1.3 * | − 0.2 |
| after 8 washes | 0 | + 2.4 ** | + 1.0 * |

\* significant difference versus reference

\*\* significant difference versus both reference and the other cellulase treatment

The results of the method of the invention (1) and of the standard full-fledged washing method (2) are found to correlate; the method of the invention is perfectly adapted to predict that, in this instance, Celluzyme [(R)] is a better performing cellulase in terms of through-the-wash fabric softening, as compared to Celluclast [(R)].

It must be noted also that the method of the invention takes about 4 hours, while the standard full-fledged washing method takes about 4 days. The advantage of the method of the invention, in terms of time, is clear.

The number of swatches used in the present example is, of course, not intended to be limiting in any manner; depending on the number of treatments and the number of replicates desired, the number of swatches used will vary; such a number will need, however, to be adapted to the capacity of the various equipments used.

Example 2

The method of the invention is used in the same way as in example 1, to evalutate the same cellulases, but the detergent composition is replaced by a triethanolamine buffer solution (0.5% in wash solution), adjusted to pH 7.5 with $NaHSO_4$ (same pH as obtained when using the detergent composition).

% $C_{14}$ CMC removal results, by Celluzyme [(R)] and Celluclast [(R)] are in line with the results obtained in Example 1

## Claims

1. A method for evaluating the performance of cellulase preparations for use in detergent compositions, comprising the steps of :
   - depositing a radiolabelled substituted cellulosic material onto a fabric substrate.
   - washing said fabric substrate with a composition containing the cellulase preparation to be evaluated.
   - determining, by radioactivity measurement, the amount of substituted cellulosic material removed from said fabric substrate, by the cellulase.

2. The method of claim 1 wherein the composition containing the cellulase preparation to be evaluated is a detergent composition.

3. The method of claim 1 wherein the substituted cellulosic material is carboxymethyl cellulose.,

4. The method of claim 1 wherein the fabric substrate is a cellulosic fabric.

5. The method of claim 1 wherein the substituted cellulosic material is labelled with carbon 14.

6. The method of claim 5 wherein the substituted cellulosic material is carboxy methyl cellulose labelled by carbon 14 on the carboxy groups.

7. The method of claim 1 wherein the radioactivity measurement to determine the removed amount of substituted cellulosic material, utilizes scintillation counting techniques.


## Patentansprüche

1. Verfahren zur Bewertung der Leistung von Cellulase-Präparaten für die Verwendung in Detergens-Zusammensetzungen, umfassend die Schritte von:
   - Ablagern eines radioaktive markierten substitutierten Zellulosematerials auf einem Gewebesubstrat,
   - Waschen des genannten Gewebesubstrates mit einer Zusammensetzung, welche das zu bewertende Cellulase-Präparat enthält,
   - Bestimmen der Menge an substituiertem Zellulosematerial, welche von dem genannten Gewebesubstrat durch die Cellulase entfernt wurde, mittels Radioaktivitätsmessung.

2. Verfahren nach Anspruch 1, worin die das zu bewertende Cellulase-Präparat enthaltende Zusammensetzung eine Detergens-Zusammensetzung ist.

3. Verfahren nach Anspruch 1, worin das substituierte Zellulosematerial Carboxymethylzellulose ist.

4. Verfahren nach Anspruch 1, worin das Gewebesubstrat ein Zellulosegewebe ist.

5. Verfahren nach Anspruch 1, worin das substituierte Zellulosematerial mit Kohlenstoff 14 markiert ist.

6. Verfahren nach Anspruch 5, worin das substituierte Zellulosematerial Carboxymethylzellulose ist, welche an den Carboxygruppen durch Kohlenstoff 14 markiert ist.

7. Verfahren nach Anspruch 1, worin bei der Radioaktivitätsmessung zur Bestimmung der entfernten Menge an substituiertem Zellulosematerial Szintillationszählverfahren verwendet werden.


## Revendications

1. Procédé pour évaluer la performance de préparations de cellulase à utiliser dans des compositions détergentes, comprenant les étapes qui consistent à :
   - déposer une substance cellulosique substituée radiomarquée sur un substrat de tissu;
   - laver ledit substrat de tissu avec une composition contenant la préparation de cellulase à évaluer;
   - déterminer, par mesure de la radioactivité, la quantité de substance cellulosique substituée éliminée dudit substrat de tissu par la cellulase.

2. Procédé selon la revendication 1, dans lequel la composition contenant la préparation de cellulase à évaluer est une composition détergente.

3. Procédé selon la revendication 1, dans lequel la substance cellulosique substituée est de la carboxyméthylcellulose.

4. Procédé selon la revendication 1, dans lequel le substrat de tissu est un tissu cellulosique.

5. Procédé selon la revendication 1, dans lequel la substance cellulosique substituée est marquée au carbone 14.

6. Procédé selon la revendication 5, dans lequel la substance cellulosique substituée est de la carboxyméthylcellulose marquée au carbone 14 sur les groupes carboxy.

7. Procédé selon la revendication 1, dans lequel la mesure de la radioactivité servant à déterminer la quantité de substance cellulosique substituée éliminée emploie des techniques de comptage à scintillation.